# EUROPEAN PATENT SPECIFICATION

(11) **EP 3 459 356 B1**
(45) Date of publication and mention of the grant of the patent: **03.06.2020**
(21) Application number: 18152812.6
(22) Date of filing: 22.01.2018
(51) Int. Cl.: A61K 8/98, A61Q 19/00, A23J 1/06, A23L 3/12, B02C 13/00, A23K 10/24, A23J 3/12

(54) **IMPROVED METHOD FOR PRODUCING BLOOD MEAL**
VERBESSERTES VERFAHREN ZUR HERSTELLUNG VON BLUTMEHL
PROCÉDÉ AMÉLIORÉ DE PRODUCTION DE FARINE DE SANG

(43) Date of publication of application: 27.03.2019
(73) Proprietor: Tessenderlo Group NV, 1050 Brussel (BE)
(72) Inventor: BELMANS, Marc, 3980 Tessenderlo (BE); DELMOTTE, Matthieu, 35000 Rennes (FR); FILLIÈRES, Romain, 27200 Vernon (FR); LOUSSOUARN, Vincent, 56930 Plumeliau (FR)
(74) Representative: Hoyng Rokh Monegier LLP

(56) References cited:
- CN-A- 1 994 319
- GB-A- 463 464
- US-A1- 2012 027 867
- BOISEN ET AL: "Prediction of the apparent ileal digestibility of protein and amino acids in feedstuffs and feed mixtures for pigs by in vitro analyses", ANIMAL FEED SCIENCE AND TECHNOLOGY, ELSEVIER, AMSTERDAM, NL, vol. 51, no. 1-2, January 1995 (1995-01), pages 29-43, XP005499283, ISSN: 0377-8401, DOI: 10.1016/0377-8401(94)00686-4

## Description

### FIELD OF THE INVENTION

The present invention relates to a method for producing blood meal. The present invention further relates to a blood meal and to uses of the blood meal in feed.

### BACKGROUND OF THE INVENTION

Besides the food grade blood which is collected in pig and cattle slaughterhouses with special means like for example hollow knives with direct injection of anticoagulant, blood is generally an under valorized by-product from slaughtered poultry, duck, pigs, cattle, sheep and the like although it is high in digestible protein content. The treatment of blood however generally reduces its nutritional value, and/or the treatment is relatively expensive. Nevertheless, treatment is necessary to obtain a storage stable powdery material. Such storage stable material is often called 'blood meal'.

Several processes are described to process blood plasma and/or anti-coagulated blood, like for example described in US2015/056363. However, the use of anti-coagulated blood requires addition of anti-coagulants in the slaughterhouses immediately after the blood has been withdrawn from the animals. A method and system for collecting blood without coagulation and in particular poultry blood is described in WO 01/08501 A1.

Anti-coagulated blood is generally separated into a plasma fraction and a hemoglobin fraction and both streams are further dried by spray drying, yielding products with very good nutritional value. However, production is complicated because of the required involvement at the slaughterhouse and spray drying has relatively high costs. Furthermore, the presence of anticoagulant often causes additional ash content or organic materials that lower the amino acid value of the product.

In case no specific measures to preclude coagulation are taken, slaughterhouses provide partly coagulated blood as by-product. This blood by-product is naturally coagulated, while still comprising dissolved proteins, like plasma proteins. This blood by-product is obtained by gathering the waste bloodstreams and this side stream generally is diluted with water due to water-cleaning. This blood by-product generally is a heterogeneous mixture of coagulated and non-coagulated blood particles, blood compounds, blood proteins, partially hemolyzed blood cells and additional water with typically about 5-18wt% solids.

This (partly coagulated) blood by product is generally processed to first obtain fully coagulated blood. This full coagulation generally is achieved by a steam treatment, which step is generally done at a blood meal production plant. Generally, the coagulated blood is thereafter subjected to a concentration step, and dried in a disc dryer, ring dryer or the like.

Continuous production for providing blood meal from coagulated blood is described in for example US3431118 and US3450537. US4067119 describes the use of a drum dryer, while GB2303042 describes the now common process of coagulation, decanting (and / or centrifuging), drying in a disc dryer and milling to particles of a size of about 1 mm. Further, it is known to dry coagulated blood in a ring or flash dryer, wherein blood particles are blown in a drying tube.

The blood meal can be used as supplement proteinaceous material in feed, such as pet food, aqua-feed and the like.

In particular for industrial applications, like aqua-feed, it is important that the nutritional value is high. Such products for example require a high protein digestibility, which can be achieved by spray drying, but at relatively high cost. Spray dried material is currently the bench mark of high quality blood meal.

### SUMMARY OF THE INVENTION

It is an object of the present invention to provide a method for producing blood meal, from raw whole blood, raw hemoglobin and/or raw plasma fractions, which is high in nutritional value and can be efficiently produced.

It is a further object of the invention to provide blood meal from coagulated blood with higher digestibility and preferably better other properties than hitherto obtainable.

This object of the invention is achieved by the method for producing blood meal comprising the subsequent steps of (i) providing an aqueous mixture comprising raw blood, preferably having a solid content between 5 wt% and 18 wt%, and (ii) increasing the solid content of the mixture to obtain a mixture having a solid content of 20 wt% or higher, preferably between 20-80 wt% and (iii) concurrently drying and grinding the resultant mixture in an air turbulence mill, to obtain dried blood meal having an average particle size (d50) between 20 µm and 0.7 mm, a d90 of below 1 mm as measured with laser diffraction using a dry powder Beckman Coulter particle size analyzer, and an ileal digestibility of 85% or higher preferably of 87% or higher, and more preferably 90% or higher.

The invention furthermore provides for a coagulated blood meal product having an average particle size (d50) between 20 µm and 0.7 mm and a d90 of below 1 mm as measured with laser diffraction using a dry powder Beckman Coulter particle size analyzer, and having an ileal digestibility of 85% or higher preferably 87% or higher and even more preferably 90% or higher, and having a moisture content of less than 15 wt%, preferably less than 10 wt%.

The process and the product of the present invention allows to achieve a number of advantages:
The efficient drying in a short time results in a product with excellent quality attributes, like for example an ileal digestibility of preferably 85% or higher or even 87% or higher like for example 90%. Generally, disc dried coagulated blood has an ileal digestibility of 60-75%, while flash, ring or drum dried material may have an ileal digestibility of up to 84%.
Furthermore, the product can have an Apparent Digestibility Coefficient (ADC) on trout of about 80% or higher based on crude protein, preferably about 85% or higher and even more preferably 87% or higher.
Furthermore, the lysine availability appears to be very high. Lysine is an amino acid which is often limiting the growth of poultry or swine. The lysine availability of the product made with the process according to the invention is generally about 92% or higher, preferably about 95% or higher relative to the total lysine content.
Furthermore, the product obtained with the process of the present invention can have a more uniform, lighter color virtually without black spots.

Also, the odor is improved (less odor) relative to conventional blood meal.

The process is furthermore efficient in that up to five operations can be performed in one relatively simple set up. In conventional processes, drying, grinding, micronizing, cooling and sieving are operations performed in different steps, while the current process allows to perform these 5 steps in one operation. Furthermore, a short residence time of a few seconds is possible.

The process can be used with anti-coagulated blood, raw hemoglobin and/or raw plasma fractions as an alternative for spray drying with the advantage that much smaller equipment is required.

The process of the present invention has the additional benefit of mitigating significantly the risk of microbiological contamination of the dried product with Salmonella and the like. The absence of dead zones in the air turbulence mill due to the turbulence created by the high speed rotation of the rotor and the flow of gas and the easy possibility of increasing - if necessary - the temperature of the flow of gas during a production run or after or before a production run are important factors to avoid product buildup or cold spots and thus prevents and mitigates microbiological contaminations.

The process does not require any special measures at the slaughterhouse, as is for example necessary for obtaining non-coagulated blood that is used for spray drying, although anti-coagulated blood, raw hemoglobin and/or raw plasma fractions could be used, mixed with (partly) coagulated blood, or as a sole supply.

### DETAILED DESCRIPTION OF THE INVENTION

### Raw materials

Raw blood can be obtained from a variety of animals, such as poultry (including chicken, turkey), duck, swine, cattle (including cows, horses, goat), sheep and the like which are handled in slaughterhouses. Mixtures of animal blood may be used.

As used in the claims, 'raw blood' may represent a major fraction of blood, like heamoglobin, plasma or mixtures of these with whole blood.

In a preferred embodiment, raw blood is derived from chicken, turkey, duck, swine, sheep or cows, or mixtures thereof.

Raw blood is generally collected from a slaughterhouse as anti-coagulated blood, or as a mixture of partly coagulated blood, dissolved proteins, partially hemolyzed bloods cells and water (together: partly coagulated blood).

Food grade blood is typically withdrawn from the animal (mainly swine and bovine) using a "hollow knife" which is inserted directly in the jugular vein of the animal and injection of the anticoagulant is made directly inside the knife or a few cm after; in other cases the blood drips into a collecting channel with continuous spraying of anticoagulants on the flow of blood.

Non-food grade blood is generally not anti-coagulated and will drip from the animal and fall directly on the floor or on a collecting pane which is periodically flushed with water to help the blood flow towards the draining point where a pump would then suck the blood and pump it to a storage tank.

Although anti-coagulated blood, raw hemoglobin and/or raw plasma fractions generally are of food quality, batches may be not up-to standard, and such anti-coagulated raw blood or fraction thereof may be used as such, or mixed with partly coagulated raw blood in the process of the present invention to provide feed additives.

Alternatively, anti-coagulated blood, raw hemoglobin and/or raw plasma fractions of food quality can be processed according to the method of the invention to produce food quality blood meal.

The amount of solids in raw blood generally is about 5 wt% to 18 wt% and typically between 8 wt% and 12 wt%, although the invention can be implemented using other solid contents.

Blood in the animal body generally has a solid content of about 17 wt% to 19 wt%. As the non-food grade raw blood normally gets diluted with water coming from the flushing and cleaning of the blood collecting and draining system in the slaughterhouse, the solid content will be below 17 wt%, generally below 14 wt%, but more likely about 12 wt% or lower. In a poultry slaughterhouse additional blood dilution water can come from the water dripping from the heads of the birds just after the water bath of the electrical stunning station.

The raw blood for use in the present invention preferably has a high protein content (generally more than 80 wt% of the dry substance), comprising at least 17 amino acids. The protein content normally is determined by measuring the total amount of nitrogen, and multiplying said total nitrogen content with the so-called Jones factor of 6.25. The result is the theoretical amount of protein. Generally, the raw blood comprises between 80 and 95% protein on solids.

Preferably, if anti-coagulated blood is processed, it can be used as is, or it can be fractionized. In certain processes, plasma is separated from a hemoglobin fraction by for example centrifugation. The hemoglobin fraction is (caused by the separation from plasma) increased in solid content relative to the raw blood, and is generally about 30-40 wt% solid. With this increase in solid content, the hemoglobin fraction can be used as a feed to the air turbulence mill, or the solid content can be further increased as described below for other blood fractions.

The plasma fraction of anti-coagulated blood after separation of the hemoglobin fraction generally is between 4-8 wt% solid, and its solid content needs to be increased, which can for example be achieved with ultrafiltration to a solid content of for example between 25-30 wt%. This mixture can further be processed as other blood mixtures described below.

Preferably, raw blood, in particular raw blood that is not anti-coagulated, is first filtered on a moving or non-moving filtration device to remove foreign materials such as animal parts (mainly feathers and heads) and crop rejects.

The solid content of the raw blood is increased to allow efficient drying in an air turbulence mill. Preferably, the solid content is increased by 10% or more, preferably by 20 wt% or more, like for example from a solid content of 18 to 28 wt% (10 wt% increase), or from 12 wt% to 32 wt% (20 wt% increase). Even more preferably, the increase in solid content is about 30 wt% or more. As will be understood from the above, raw blood not necessary reflects the whole raw blood composition, but may be a fraction thereof.

In a preferred embodiment, the solid content of the blood is increased to between 20 and 60 wt%, preferably between 40 to 60 wt%.

In a first preferred embodiment, the raw blood is treated to (further) coagulate the blood and subsequently to increase the solid content.

The coagulated blood so obtained is referred to as fully coagulated blood, or clotted blood. In practice, not all of the protein content is coagulated, yet the amount of coagulated or denatured proteins is substantially higher than in the partly coagulated blood mixture or in anti-coagulated blood.

The coagulation treatment may be effected mechanically, with heat, steam and/or use of chemicals. Chemical coagulation of industrial animal blood can be effected by using for example aluminum sulfate, zinc sulfate, methanol, and acetone (see Andrew L. Ratermann, H. Wayne Burnett, Vaughn Vandegrift; J. Agric. Food Chem., 1980, 28:438-441).

Preferably, the coagulation is effected mechanically and/or with heat treatment. Even more preferable, the partly coagulated blood is further coagulated by direct contact and mixing with live steam.

The further coagulation also serves to homogenize the partly coagulated blood mixture.

The clotted blood is preferably dewatered to increase the solid content of the clotted blood to about 20-60 wt% solids on dry basis, preferably 40-60% solids on dry basis. Water can be removed by evaporation or mechanically by pressing blood clot over a sieve, centrifuging or using ultrafiltration. Clotted blood preferably is centrifuged to remove water from the clotted blood. Evaporation can for example be done at reduced pressure for example at a temperature of 30-60 °C.

It is furthermore possible to further increase the solid content by drying in conventional drying equipment up to for example 80 wt% solids or more preferably up to the point that the digestibility of the semi-dried product will start to decrease significantly. Such moisture content will vary depending on the type of pre-drying equipment that is being used. In a preferred embodiment, the solid content is increased to about 60%.

The residual water commonly referred to as blood water resulting from for example centrifuging may contain 1 to 5 wt% or typically 1 to 4 wt% solids. The blood water is a waste stream that may be concentrated to for example 15-40 wt% solid by using ultrafiltration among other means, and the concentrated stream can be for example spray dried, but preferably is recirculated into the production process to be dried in the air turbulence mill. The blood water alternatively can be considered a waste stream that can be treated in a waste water treatment plant.

Preferably, dried blood meal material is back-mixed with the concentrated blood water stream and this mixture can be supplied to the air turbulence mill directly, or this can be mixed with the main clotted blood stream before entering the air turbulence mill.

When using clotted blood, it may be useful to apply back-mixing of dried blood meal product into the feed stream of the air turbulence mill to (further) increase by this way the solid content of the feed to the air turbulence mill.

In a second, alternative, preferred embodiment, the raw blood as obtained from the slaughterhouse may be homogenized and is increased in solid content without the need of further coagulation with for example live steam.

Homogenization is particularly useful when partly coagulated blood is processed, as that is heterogeneous material. Homogenization can be achieved in a vessel or when transporting the blood mixture to the air turbulence mill by general available homogenization equipment like a mixer. Preferably, in the homogenization step large particles of a size of more than a 1 cm are reduced in size.

The increase in solid content of this raw blood can be achieved for example by back-mixing blood meal into the supply stream to the air turbulence mill and/or by evaporation of water from the mixture.

The increase in solid content preferably is at least partly achieved by back-mixing blood meal into the supply stream of the blood mixture before introduction into the air turbulence mill. The increase in solid content can additionally be achieved by evaporation of water at reduced pressure. It is preferred that the temperature during these treatments remains at about 100 °C (boiling water temperature) or lower, preferably 80 °C or lower, preferably about 70 °C or lower, and even more preferably about 60 °C or lower.

In general, therefore, the raw blood mixture obtained from slaughterhouses may be homogenized and is increased in solid content from below 14 wt%, typically from below about 10 to 12 wt%, to at least 20 wt%, and more preferably to about 40 wt% or more, like up to 50 wt% by removal of water and/or by adding dried blood meal and/or a concentrated blood mixture to the feed stream of the air turbulence mill.

If water is removed from the raw blood mixture by for example evaporation as part of the step to increase the solid content, the solid content of the stream fed to the air turbulence mill, before adding a back-mixing stream, is generally between about 20-40 wt%, like for example about 30 wt%.

If blood meal is added to the raw blood stream as the sole measure for increasing the solid content, the solid content of the mixed stream to the air turbulence mill generally will be about 50 wt% or less.

The homogenized at least partly coagulated blood, which is increased in solid content to preferably above 40 wt% can subsequently be dried as a mixture in the air turbulence mill.

In one embodiment of the invention, the clotted blood mixture is supplied to the air turbulence mill while having a moisture content of preferably between 40-60 wt%.

In another embodiment, the optionally homogenized raw blood mixture, after increased solid content, is supplied to the air turbulence mill at a solid content of between 20-55 wt%, preferably at 30-50 wt% solid content.

### Drying process and the air turbulence mill with accompanying parts

The blood mixture with increased solid content will be dried and ground according to step (iii) of the present invention.

Generally, the blood mixture is dried to a moisture content of about 15 wt% or lower. In a preferred embodiment of the invention, the blood mixture is dried to a moisture content of about 10 wt% or less, preferably about 8 wt% or less. Drying to an amount of water lower than about 4 wt% generally is not necessary, but would not harm. Drying is most preferably performed such that the dried blood meal has a moisture content of about 5 to 7 wt%. Drying results in a storage stable product.

In a preferred embodiment, the dried blood meal is divided in a product stream and a recycle stream.

The product leaving the air turbulence mill may contain a relatively small fraction of ultrafine product, too coarse material which may be ground, or otherwise unsuitable material, which preferably is separated in a classifier. Unsuitable material preferably is recycled (back-mixed) into the supply stream of the air turbulence mill.

Preferably, if larger amounts of back-mixing is useful, part of suitable product is used as a recycle stream for back-mixing into the feed of the air turbulence mill.

The recycle stream (the back-mixing of dried blood meal) can be used according to the invention to increase the solid content of the feed stream to the air turbulence mill.

Drying is an important step for the final quality of the blood meal.

It appears that common drying techniques for clotted blood cause the digestibility to be reduced.

In the preferred embodiment of the invention, the drying of step (iii) is performed at about atmospheric pressure while forming small particles, such that drying is very efficient. The atmospheric pressure includes slight vacuum, which is often used to aid the flow of the gas and transport of the powder. The powder may be retrieved from a dry cyclone by means of an air lock rotating valve. The pressure before the mill can be -5 to -8 mbar and after the mill between -30 and -50 mbar.

For example, drying in a conventional disc dryer generally yields an ileal digestibility of about 60-75%. Ring, flash or drum dried blood meal (also denoted as flash dried meal) can have an ileal digestibility of around 84%.

Preferably, the concentrated blood mixture resulting from step (ii) is dried with a method allowing low heat damage, such that the reduction of the digestibility during drying is limited and that the blood meal is characterized by an ileal digestibility remaining higher than 85%, preferably about 87-93%, like for example 90-92%.

Preferably, the concentrated blood mixture resulting from step (ii) is dried with a method allowing low heat damage, such that the blood meal preferably has an Apparent Digestibility Coefficient (ADC) on trout of about 85% or higher based on crude protein.

For example, drying in a conventional disc dryer generally yields blood meal with an ADC on trout of 65% or lower based on crude protein.

To obtain such material with low heat damage, the inventors of the present invention discovered that drying is to be carried out with a gas flow while at the same time grinding the material allowing a very short residence time.

The present inventors found that further improvement of the in vitro digestibility and nutritional characteristics of blood meal was achievable by using an air turbulence mill, because the small particles that result from the grinding action aid in quickly drying the concentrated blood mixture and therefore limits the exposure of the product to heat.

Hence, according to the present invention, the increased-solid blood mixture resulting from step (ii) is concurrently dried and ground with a gas stream, generally air (that may be low in oxygen), using an air turbulence mill. The gas may also be superheated steam. The air turbulence mill has the benefit of a fast grinding and drying-effect, and the use of an air turbulence mill according to the invention results in drying and simultaneously milling or grinding the blood mixture by introducing the material to dry and a flow of gas, generally air, into a high speed rotor in a confined chamber.

An air turbulence mill generally comprises a chamber (stator) with appropriate inlets and outlets for product and stream(s) of gas in which a rotating member (rotor) is mounted with stacks of impacting devices which rotating member can rotate at high speed. The inner walls of the stator are preferably lined with impacting members, like corrugated sheets, in order to increase the efficiency of the grinding with additional friction and shear forces. The rotor generally is placed vertically relative to the outlet.

Several types of air turbulence mills exist. They are generally referred to as turbulent air grinding mills or vortex air mills. The present invention contemplates the use of all of them under the notion of "air turbulence mill". It is preferred to use vertically positioned rotors, as these appeared to use less energy.

Air turbulence mills, such as those known in the art from Atritor (Cell Mill), Hosokawa (Drymeister), Larsson (Whirl flash), Jäckering (Ultra Rotor), Rotormill or Görgens Mahltechnik (TurboRotor) may be used for drying and grinding in the present invention. Some of such air turbulence mills are described in e.g. US4747550 and WO1995/028513.

The air turbulence mill may comprise a classifier, which causes a separation of larger and smaller particles. The use of a classifier allows the larger particles to be returned to the grinder, while smaller particles are left through for further processing. In another embodiment, two or more grades of particulate blood meal are produced, with differing particle sizes and bulk properties by having two outlets out of the classifier.

The drying is performed with a stream of gas into a high speed rotor. The gas stream generally is air, that may be low in oxygen, but it can also be superheated steam. The inlet temperature generally ranges between 20 °C and 500°C, preferably between 20 °C and 450 °C and even more preferably 20 °C and 180 °C. The higher end of the temperature may require careful processing and/or may require lower amounts of the heated gas to be used. It would for example be possible to use the heated gas at a temperature of 450 °C and a second gas stream at room temperature if high gas velocities are required.

The outlet temperature of the air generally is below 100 °C, preferably below 90 °C. The temperature of the inlet-gas may be lower in case the concentrated blood mixture feed has a higher temperature.

The flow of the air generally is 5 m³/h per kg of fed material or higher, preferably 7 m³/h per kg fed material. Generally, the amount is 50 m³/h or less, preferably 30 m³/h per kg fed material or less. Suitable, most preferred, amounts are for example between 5 and 30 m³/h, such as between 7-20 m³/h per kg of fed product.

The gas flow can be fed into the mill directly with the feed material, or indirectly, wherein the concentrated blood mixture is fed on one place, and the gas stream is fed into the air turbulence mill separately in one or several other places. Product can for example be fed directly into the side of the lower part of the mill or mixed with the air flow just before the mill. Alternatively, the product feed can be directly fed through an inlet flange on the side of the mill body.

The air turbulence mill used in the present invention preferably comprises a confined chamber (stator) with appropriate inlets and outlets for the product and the stream of gas in which a vertically placed shaft (rotor), mounted with stacks of cutting and impacting devices such as blades, discs, plates and the like, rotates at high speed. The inner walls of the stator can be lined with corrugated sheets in order to increase the efficiency of the grinding with additional friction and shear forces.

The rotor generally rotates with a tip speed of about 20 m/s or higher, more preferably of about 35 m/s or higher, even more preferably of about 50 m/s or higher. Generally, the speed is about 250 m/s or lower, preferably about 150 m/s or lower. A suitable speed is for example about 75 m/s. Generally, the tip speed is about 80 m/s or higher, preferably about 110 m/s or higher.

The grinder may produce substantial heat. Furthermore, the incoming wet concentrated blood mixture may be at a temperature above room temperature. If useful, the gas stream can be heated, for example by direct heating in a gas burner (which also causes the oxygen level to be reduced, which lowers ignition hazard), or by indirect heating through a heat exchange with steam or hot oil. The gas stream may also be superheated steam.

The air turbulence mill comprises one or more inlets for a gaseous flow. One or more of these gas streams may be heated. In case one stream of gas is heated, it is preferably heated to a temperature of about 50 °C or higher.

The gaseous flow can be introduced in different ways. Generally, the main gas stream is introduced at the bottom of the air turbulence mill. This inlet may be the same inlet as the wet product inlet. In such case, the gas flow is generally used to transport the product. A second gas stream can be used to influence the milling and flow behavior of the mill. In particular in case the product is not easy to transport with a gas flow, it can be introduced directly into the mill through a screw or pump.

To maintain a high digestibility of the blood meal the average residence time in the air turbulence mill is preferably short, like less than 10 sec, preferably below 5 seconds, more preferably below 2 seconds, even more preferably below 1 second. The low average residence time of the material to dry in the mill allows efficient drying while only a relatively small increase in temperature of the coagulated blood material is observed. In case a classifier is used, the average residence time will be higher, but the time that any powder actually is in the grinder remains preferably below 10 sec, and even more preferably below 5 sec.

Preferably the temperature of the blood meal coming out of the air turbulence mill is at a temperature range between about 30°C and 90 °C, more preferably between about 40°C and 80 °C, even more preferably between about 45°C and 75 °C.

The gas flow leaves the air turbulence mill, optionally through a classifier, with the dried product. The dry product in the form of small particles is separated from the gas stream, which separation is generally done in one or more cyclones, preferably one or two cyclones or by means of a bag filter or combinations of both.

It is possible to further classify the resultant powder leaving the cyclone, like for example on a horizontal sieve for screening oversized, large particles and/or for removing dust. It is furthermore possible to produce different grades of blood meal, with smaller and larger particle sizes.

Reject of the sieve (oversized particles and/or dust) preferably is reintroduced in the feed for further treatment in the air turbulence mill. Mixing of the reject with the wet feed material (also referred to as "back-mixing") can improve the feeding operation and overall efficiency of the drying and grinding. As described above, the reject can be mixed with a concentrated waste stream which is obtained by increasing the solid content of for example blood water, and thereafter mixed with the primary blood stream.

Depending on the feed of the air turbulence mill, it may be useful to allow substantial back-mixing, like for example back-mixing 40-80 wt% of the resultant dry product to the feed to increase the solid content of the feed, while improving the overall product characteristics.

Therefore, more generally, it is preferred to have blood meal back-mixed into the stream comprising raw or coagulated blood provided to the air turbulence mill. The amount of back mixing may depend on the solid content of the stream supplied to the air turbulence mill. Generally, the amount of back-mixed material will be between 1 wt% and 90 wt% (of the dried material), preferably between 2-80 wt%. The low back-mixing values preferably are used if only off-spec material is to be back-mixed. In order to have a substantial increase (more than e.g. 5%) in solid content, it is preferred to have back-mixing ratios of 5-90%, more preferably 10-80 wt%.

In a preferred embodiment, if the solid content of the stream supplied to the air turbulence mill which (without back-mixing) is about 20 wt% or higher, preferably 30 wt% or higher, the amount back-mixed material is preferably such, that the supply to the air turbulence mill will be about 40 wt% solid content or more. Hence, for example, back-mixing amounts of about 40 wt% to about 90 wt% are suitable if the supply stream contains about 20 wt% solids, and between about 10 wt% to about 70 wt% if the stream contains about 30 wt% solids.

The amounts are given as wt% of dry product, back-mixed into the supply stream. It is also possible to give the back-mixed ratio based on solid content of the supply stream (before back-mixing). With a stream of about 20 wt% solids, the back-mixing could be about 200-400%, preferably 200-300%, while with a supply of about 30 wt% solids, the back-mixing ratio could be between about 50% and 200%.

Preferably, classification is done over a sieve (or other classification device) with the cut off of 1 mm or lower, preferably 800 µm or lower. Classification can for example be done over a sieve with a cut off of 300 µm, 500 µm or 900 µm.

Furthermore, the flow of air entering the air turbulence mill can be adjusted to influence the residence time and/or the size of the particles. For example, the flow of air directly influences the residence time in the chamber and contact time with the milling device; the higher the flow of air the shorter the residence time and therefore the bigger the particles, and vice-versa the lower the flow of air and the smaller the particles. The size of the particles is further influenced by a classifier and/or by adequate impacting and cutting devices as well as adequate surface profiles of the inner walls. The skilled person will be able to balance the air turbulence mill to provide particle sizes as required.

### The resultant blood meal

The blood meal material is further used as a dry product with a moisture content of 10 wt% or less (i.e. blood meal).

The material comprises preferably less than 8% of moisture. Most preferable, the dried material has a moisture content of between 5 and 7 wt%.

Blood meal according to the present invention generally contains about 85 wt% to about 98 wt% protein on dry weight.

Advantageously, the blood meal produced in accordance with a method of the present invention may provide a valuable source of protein and/or source of amino acids in animal feed. For example, blood meal can provide a source of one or more of the following amino acids: methionine, cysteine, lysine, threonine, arginine, isoleucine, leucine, valine, histidine, phenylalanine, glycine, serine, proline, alanine, aspartic acid, tyrosine, tryptophan and glutamic acid. The high digestibility makes the product more effective as a feed additive than clotted blood meal of the prior art.

The ileal digestibility is 85% or higher, preferably 87% or higher, and even more preferable, 90% or higher.

The pepsin digestibility as determined by ISO 6655 (August 1997) of all blood meals is relatively high (>95%), and therefore this analytical method does not differentiate sufficiently different quality grades of blood meal. The ileal digestibility is more sensitive to processing.

The blood meal according to the present invention shows a high digestibility in in-vivo experiments on cecectomized roosters and on trout.

Furthermore, the bioavailability of lysine is also very high in the products obtained with the process of the present invention.

The clotted (or coagulated) blood meal product obtained with the process according to the invention is believed to be a novel product. The clotted blood meal shows, like other dried fully coagulated blood products, sedimentation of most of the product within 24 hr after being dispersed in water, whereas the spray dried non-coagulated product remains largely dispersed in that time period, and therefore exhibits a non-sedimentation behavior in that time period.

The invention provides for a coagulated blood meal having an average particle size (d50) between 20 µm and 0.7 mm and a d90 of below 1 mm as measured with laser diffraction using a dry powder Beckman Coulter particle size analyzer, and the product has an ileal digestibility of 85% or higher preferably 87% or higher and even more preferably 90% or higher.

The bioavailable lysine content of the coagulated blood meal product preferably is 92% or higher, even more preferably 94% or higher relative to the total lysine content.

The Apparent Digestibility Coefficient (ADC) on trout of the coagulated blood meal product preferably is 80% or higher, even more preferably 85% or higher, and most preferably 87% or higher, relative to the crude protein.

The blood meal comprises at least 17 amino acids, preferably at least 18 amino acids, such as lysine.

Preferably, the amount of lysine is about 7 wt% or more relative to the total protein content.

The blood meal is rich in haem iron which is biologically the most available form of iron.

The powder properties described below are related to all the products obtainable with the process of the present invention, and therefore apply to blood meal obtained from coagulated blood, anti-coagulated blood, and/or a mixture of both. It also applies to the hemoglobin fraction of the whole blood and/or the plasma fraction of the whole blood and/or any mixtures of hemoglobin fraction, plasma fraction, coagulated blood and anti-coagulated blood.

The dried and ground material leaving the air turbulence mill is generally in the form of particles of which more than about 99 wt% is smaller than a few mm, like for example smaller than about 2 mm, preferably smaller than 1 mm. Generally, more than about 95 wt% is larger than about 8 µm, in order to have a free flowing powder that can easily be handled and further processed into for example formulated pet foods and animal feeds.

The average particle size of the product after classification (defined as d50; 50% of the volume fraction of the particles is larger, and 50% is smaller), as measured by laser diffraction on a Beckman Coulter particle size analyzer using standard software, is between 20 µm and 0.7 mm, preferably between 20 µm and 500 µm, more preferably between 50 µm and 300 µm. For example, the average particle size is about 75 or about 150 µm.

The d90 is below 1 mm, more preferably below 0.7 mm. The d10 preferably is above 10 µm, and more preferably above 15 µm.

The above described sizes are highly advantageous. Steam coagulated blood dried in a conventional disc or drum dryer has at the outlet of the dryer a coarse heterogeneous particle size distribution with a substantial amount of particles with a size of above 1.8 mm and quite often above 2 mm or higher necessitating further grinding and sieving equipment and therefore additional footprint requirements and additional dust emissions issues. This makes such equipment a less attractive process set up.

The particle size distribution of the blood meal of the present invention is relatively homogeneous. For example, the d90 divided by the d10 is about 12 or less, preferably about 10 or less, while the d90 is 1 mm or lower.

The blood meal obtained with the method in accordance with the present invention is in the form of a powder with powder characteristics preferably such, that the powder has good flow properties, packaging characteristics as well as good dosing characteristics to formulate pet foods and animal feeds.

The Hausner ratio of the powders according to the invention (tapped density divided by poured bulk density) is about 1.06-1.18, i.e. preferably below about 1.2, which is considered an indication for good flowability.

In a preferred embodiment of the invention the blood meal has a poured (bulk) density of about 0.35 g/cm³ or higher, more preferably of about 0.45 g/cm³ or higher, even more preferably of about 0.5 g/cm³ or higher. Generally, the poured density will be about 0.7 g/cm³ or lower, like for example about 0.6 g/cm³ or lower.

In a further preferred embodiment of the invention the blood meal has a tapped bulk density of about 0.4 g/cm³ or higher, more preferably of about 0.5 g/cm³ or higher and even more preferably 0.55 g/cm³ or higher. Generally, the tapped density will be about 0.75 g/cm³ or lower, like for example 0.65 g/cm³ or lower.

In a further embodiment, the blood meal may be pelletized to increase its density to optimize volume and transport cost.

Moreover the blood meal obtained in accordance with the process as described here has a significantly milder odor compared to conventional disc dried blood meal. The high turbulence, the very short residence time, the absence of contact with hot surfaces (unlike in a disc dryer) and the absence of deposits on the inside walls (unlike in a spray dryer and unlike in the ducts of a ring dryer) avoids the formation of burnt particles and odorous compounds.

The drying and grinding in an air turbulence mill has the further advantage that the color of the material is lighter and more homogeneous than classically dried material. In particular virtually no black spots are present.

An antioxidant and/or an anticaking agent can be blended into the blood meal to improve respectively its oxidation stability and its flowability.

In a preferred embodiment the antioxidant is added as a liquid or a solid and mixed into the concentrated blood mixture prior to the turbulence mill in order to have a homogeneous incorporation and to avoid undesired spots and agglomeration if such incorporation was done on the finished powder. When back-mixing is performed using dry blood meal and therefor with dry product that contains already antioxidant, the amount of antioxidant added in the fresh wet stream is adjusted accordingly.

Before or after drying, some amino acids may be added to the blood meal. In particular, it can be useful to add isoleucine, cysteine and/or methionine, or digestible proteins containing these amino acids in relatively large amounts, as the amount of these amino acids is relatively low in blood meal. Generally, the added amounts will be about 20 wt% or less, preferably about 10 wt% or less.

The blood meal can be packed in small bags, big-bags or other bulk containers. The dry blood meal can be packed and shipped in any kind of Bulk Container, big bag or other container.

The blood meal may be used as feed, as feed supplements, such as in pet food and/or in aquaculture feeds. The material may be used in powder form or may be converted to larger dosing units in the form of granules, flakes and the like using conventional processing techniques. The blood meal can be used as carrier for other ingredients, and/or can be used as extender.

When used in the preparation of a feedstuff, the blood meal produced in accordance with the present invention may be used in conjunction with one or more of: a nutritionally acceptable carrier or extender, a nutritionally acceptable diluent, a nutritionally acceptable excipient, a nutritionally acceptable adjuvant or a nutritionally active ingredient. The blood meal may itself be a carrier or extender for other functional ingredients such as flavoring agents, palatants and attractants.

The process of the present invention can be easily applied in conventional plants that treat at least partly coagulated blood, anti-coagulated blood, fractions thereof and/or mixtures thereof, because the air turbulence mill with the optional classifier, the cyclone and the air supply occupy substantially less space than a conventional disc dryer or other conventional dryers with ancillary equipment. Hence, the present invention also relates to a method of retrofitting a blood meal plant by replacing conventional drying equipment with an air turbulence mill and a cyclone with ancillary equipment or by adding such an air turbulence mill after the conventional drying equipment and running the conventional equipment to a higher residual moisture content and finishing the drying in the air turbulence mill.

### MEASUREMENT METHODS

The following methods were used in the examples, and are suitable as methods to measure the parameters stated in the description and the claims:
Weight percentage (wt%) moisture: moist material is dried overnight in a vacuum stove at reduced pressure and with a siccative. The material is weighed before and after the drying step, and the amount of moisture is calculated using the initial measured weight as 100% while assuming that all the volatile material is water.

Solid content is defined as solids remaining after removing the water as described for wt% moisture.

Sedimentation test: 3 gram of blood meal is added to 100 ml water in a transparent beaker at room temperature and stirred for 1 min. The beaker is left to stand for 24 hr. Thereafter, a picture is taken, and it is determined whether a clear sediment is visible, or whether the liquid is homogeneously dark.

Pepsin digestibility is measured according to ISO 6655 (August 1997) using as per the said mentioned norm a pepsin concentration of 0.02% in hydrochloric acid.

Ileal digestibility (also referred to as Boisen digestibility) is measured according to the methods described by S. Boisen in "Prediction of the apparent ileal digestibility of protein and amino acid in feedstuffs for pigs by in vitro analysis", Animal Feed Science Technology, 51, pp.29-43 (1995) and further described in "In vitro analysis for determining standardized ileal digestibility of protein and amino acids in actual batches of feedstuffs and diets for pigs"; Livestock Science, 309 : pp.182-185 (2007).

Particle size distribution is measured with laser diffraction on a Beckman Coulter particle size analyzer - dry powder system. Standard software of the manufacturer was used. The results are described as d10, d50, d90 etc., which relates to the volume fractions.

The poured and tapped density are measured on a Jolting Volumeter Type STAV II from Engelsmann. According to the brochure of the manufacturer, the apparatus serves to determine the volumes before and after tamping, the compaction as well as the tapped density according to the European Pharmacopoeia, DIN ISO 787 part 11, ISO 3953, ISO 8967 and ASTM B 527-93.

Lysine was measured in accordance with ISO 5510:1984, measuring both bioavailable lysine and total lysine in order to determine the ratio of the two.

Apparent Digestibility Coefficient (ADC) experiments were performed on rainbow trout using a triplicate model test (3 tanks of 15 trout). The water of the tanks was regulated at 17°C +/-0,5 °C, the oxygen level was controlled and maintained at 9.5 ppm at the inlet, nitrogen was removed over a biological filter and the fish were exposed to a photoperiod of 12 hours. Fish were fed twice a day ad libitum. The feeds were formulated by mixing 20% of the ingredient to be tested with 80% of a reference diet containing fish meal (75%), starch, wheat gluten, a premix of minerals and vitamins and Yttrium as inert marker; the mixture was extruded in 4 mm pellets using a double screw Clextral extruder and fish oil was incorporated on the pellets under vacuum. Trout had an average starting weight between 100 and 120 grams. The tanks were equipped with an automatic faeces collection system. Faeces were collected only after one week in order to acclimate the fish to their tank environment and to their diets in order to stabilize the ADCs. The faeces were collected once a day before the morning feed, frozen and later freeze dried prior to analysis. The ADCs were calculated using the indirect method according to the formula of Maynard and Loosli (Animal Nutrition, 6th edition, McGraw Hill, New-York, 1969).

Cecectomized rooster assay: The in-vivo digestibility of crude protein, dry substance and amino acids were carried out by a specialized institute using a two replication model. Two groups of three cecectomized roosters were fasted during 24 hours and then force fed a precise amount of a formulated feed containing the dry blood sample to be analyzed. The total collection of excreta including endogenous losses was done over a period of 48 hours. The excreta were cleaned to remove foreign bodies like feathers and further frozen, freeze-dried and homogenized. The digestibility coefficients were then calculated by comparing the levels of crude protein, dry substance and total amino acids measured in the dry blood samples and in the freeze-dried excreta after correction for endogenous losses.

Further modifications in addition to those described above may be made to the materials and methods described herein without departing from the spirit and scope of the invention.

Accordingly, although specific embodiments have been described, the following are examples only and are not limiting upon the scope of the invention.

### EXAMPLES

Raw partly coagulated blood was obtained from a slaughterhouse. It comprised >90% poultry blood. The solid content was about 10%.

The raw partly coagulated blood was further coagulated with live steam (circa 150 kg of steam per ton of partly coagulated blood), and the clotted blood was concentrated in a centrifuge to obtain clotted blood mixture with about 40 wt% solids.

The clotted blood mixture was dried in an air turbulence mill (JÄCKERING, model Ultra Rotor III a), with a feed of 200 kg/h of concentrated blood mixture. The clotted blood mixture was dried and ground in a number of runs. The air turbulence mill was operated with an inlet gas (air) at a temperature of 160 °C. The amount of gas was between about 15 and 30 m³/hr*kg. The tip speed of the rotor was between 80 and 90 m/s.

The average residence time of the product was estimated to be less than about 1 sec. During drying and grinding, it was estimated that the blood meal product did not reach temperatures higher than about 80 °C, because the temperature of the air at the outlet of the air turbulence mill was about 90°C. The product was screened over a 300 µm sieve, and the fraction with smaller particle size was further characterized as described below. The fraction (about 2-5 wt%) with larger particle size was in one trial fed-back into the feed of the air turbulence mill; this run was operating smoothly.

The product so obtained was compared with conventional dried (in a disc dryer) blood meal; in a conventional ring dryer, and also compared to spray dried blood meal. It must be recognized that spray dried material is made from anticoagulated blood, as otherwise the spray nozzles would clog.

Comparisons were made on a number of characteristics. As can be derived from the following table:
- Ileal (Boisen) digestibility is higher for the product made with the process according to the invention in comparison with conventionally dried products of clotted blood.
- The in-vivo digestibility on cecectomized roosters is much better for the product according to the invention, in comparison with conventionally dried clotted product, while it is virtually equal with spray dried blood meal.
- The Apparent Digestibility Coefficient (ADC) on trout based on crude protein is much better for the product made with the process according to the invention in comparison with conventionally dried products of clotted blood.
- The biologically available lysine, which is an important measure for biologically available amino acids, is substantially higher in the product according the invention, also in comparison with spray dried product.
- The product obtained with the process of the invention can have a particle size distribution as fine as obtained with spray drying. A narrower particle size distribution is possible with the process according to the invention. It should however be noted, that it is easy to obtain coarser material by allowing classification at for example 1 mm instead of 300 µm.
- The sedimentation test shows that the blood meal from clotted blood exhibits clear sedimentation unlike spray dried blood meal.

Furthermore (not shown in the table), the color of the product made with the process according to the invention is lighter and more homogeneous in comparison with conventionally dried products of clotted blood. Also, the product obtained with the process of the invention has a significantly milder odor (neutral to mild odor) compared to conventionally dried products of clotted blood.

The process of the present invention allows to obtain very high quality blood meal, with even a biologic available lysine content substantially higher than the best quality blood meal to date (spray dried blood meal).

The process using an air turbulence mill is substantially cheaper and simpler to operate than spray drying, and allows to achieve a quality which is substantially comparable to if not better than a spray dried product, which is very advantageous.

| | disc dried (commercial) | ring dried (commercial) | spray dried (pilot test) | spray dried (commercial) | according present invention |
|---|---|---|---|---|---|
| | | | | | |
| Ileal/Boisen digestibility (%) | 61,3 - 82,1 | 84,0 | 97,0 | 85,7 - 89,5 | 90,2 - 93,7 |
| | | | | | |
| in-vivo digestibility roosters (%) | | | | | |
| on dry matter | 79,4 | 78,6 | 84,8 | | 89,6 |
| on energy | 76,6 | 83,9 | 92,6 | | 91,0 |
| on crude protein | 79,0 | 86,2 | 92,5 | | 91,9 |
| | | | | | |
| in-vivo digestibility trout (%) | | | | | |
| on dry matter | 62,7 | 84,9 | | | 89,2 |
| on energy | 61,7 | 80,9 | | | 86,6 |
| on crude protein | 64,8 | 84,2 | | | 89,1 |
| | | | | | |
| biological lysine availability (%) | 91,0 | | | 88,6 | 96,0 |
| | | | | | |
| particle size distribution | | | | | |
| d10 | 67,2 | | | 18,6 | 20,1 |
| d25 | 119,5 | | | 33,4 | 34,1 |
| median ; d50 | 227,7 | | | 59,8 | 53,2 |
| d75 | 512,5 | | | 108,0 | 79,1 |
| d90 | 877,1 | | | 159,0 | 112,7 |
| mean | 373,6 | | | 75,3 | 60,6 |
| d90/d10 | 13,1 | | | 8,5 | 5,6 |
| | | | | | |
| sediment test | clear sediment | clear sediment | | homogeneously dark | clear sediment |
| | | | | | |
| some of the amino acids (%) | | | | | |
| lysine | 8,85 | 8,58 | | 7,9 | 8,37 |
| threonine | 4,71 | 5,32 | | 5,3 | 4,62 |
| tryptophan | 0,94 | 2,09 | | nd | 1,89 |
| isoleucine | 3,25 | 3,98 | | 3,94 | 3,11 |
| leucine | 11,34 | 10,3 | | 9,55 | 10,8 |

## Claims

1. Method for producing blood meal comprising the subsequent steps of (i) providing an aqueous mixture comprising raw blood, preferably having a solid content of between 5 and 18 wt% and (ii) increasing the solid content of the mixture to obtain a mixture having a solid content of 20 wt% or higher, preferably between 20-80 wt% and (iii) concurrently drying and grinding the resultant mixture in an air turbulence mill, to obtain blood meal having an average particle size (d50) between 20 µm and 0.7 mm, a d90 of below 1 mm as measured with laser diffraction using a dry powder Beckman Coulter particle size analyzer, and an ileal digestibility as measured according to the method described by Boison in Animal Feed Science Technology, 51, pp.29-43 (1995) and in Livestock Science, 309 : pp.182-185 (2007), of 85% or higher preferably of 87% or higher, and more preferably 90% or higher.

2. Method according to claim 1, wherein the aqueous mixture of step (i) is subjected to a coagulation step, preferably using live steam, and wherein subsequently an increased solid content is obtained by removal of part of the water to obtain a clotted blood mixture having a solid content of between 30-70 wt%, preferably 40-60 wt%.

3. Method according to claim 1, wherein the aqueous mixture comprises raw blood and wherein the mixture has a solid content below 18 wt%, or below 12 wt%, wherein the mixture is optionally homogenized and is increased in solid content to a solid content of 30 wt% or more by removal of water and/or by adding dry blood meal and/or concentrated blood during step (ii), preferably at least part of the increase in solid content is achieved by back-mixing dry blood meal into the mixture.

4. Method according to claim 3, wherein the amount of back-mixed blood meal is between 2 wt% and 90 wt% (of the dried material), preferably between 10 wt% and 80wt%.

5. Method according to any one of the preceding claims, wherein the solid content in step (ii) is increased 10 wt% or more, preferably 20 wt% or more.

6. The method according to any one of the preceding claims wherein concurrently drying and grinding is performed at a temperature such that the material being dried and ground remains at a temperature of 90 °C or below, preferably of 80 °C or below and more preferably below 60°C.

7. The method according to any one of the preceding claims, wherein the air turbulence mill comprises a chamber with appropriate inlets and outlets for product and stream(s) of gas in which a rotating member is mounted with stacks of impacting devices which rotating member can rotate at high speed, and wherein preferably the inner walls of the stator are lined with impacting members, wherein the rotating member rotates at a tip speed between 35-250 m/s.

8. The method according to any one of the preceding claims, wherein the air turbulence mill comprises an internal or external classifying device allowing recirculation of coarse material into the mill.

9. The method according to any one of the preceding claims, wherein the air turbulence mill is operated with a flow of gas, preferably air with optionally a lowered oxygen content, at a temperature between 20 °C and 500°C, preferably between 20°C and 450 °C and wherein the gas flow is between 5 and 50 m³/hr per kg feed, which flow may be adjusted to influence the particle size of the dry blood meal, and wherein the residence time is less than 10 sec.

10. The method according to any one of the preceding claims, wherein the blood is obtained from animals, chosen from poultry, duck, swine, cattle, including cows, horses, goat, and sheep.

11. Coagulated blood meal having an average particle size (d50) between 20 µm and 0.7 mm, a d90 of below 1 mm as measured with laser diffraction using a dry powder Beckman Coulter particle size analyzer, and an ileal digestibility, as measured according to the method described by Boison in Animal Feed Science Technology, 51, pp.29-43 (1995) and in Livestock Science, 309 : pp.182-185 (2007), of 85% or higher preferably 87% or higher and even more preferably 90% or higher, and a moisture content of 10 wt% or less, preferably 5-8 wt%.

12. The blood meal according to claim 11, wherein the dry blood meal has a bioavailable lysine content as measured according to ISO 5510:1984 of 92% or higher, preferably 94% or higher.

13. The blood meal according to any one of claims 11-12, wherein the Apparent Digestibility Coefficient as measured according to the method defined in the description of the dry blood meal on trout is 80% or higher based on crude protein, preferably 85% or higher, and more preferably 87% or higher.

14. The blood meal according to any one of claims 11-13, wherein the blood meal has an average particle size (d50) of between 20 µm and 0.5 mm measured by laser diffraction using a dry powder Beckman Coulter particle size analyzer, preferably of between 50 µm and 300 µm.

15. The blood meal according to any one of claims 11-14, wherein the blood meal has a d90 particle size of 0.7 mm or below measured by laser diffraction using a dry powder Beckman Coulter particle size analyzer, and/or wherein the blood meal has a d10 particle size of 10 µm or above, preferably of 15 µm or above, as measured by laser diffraction using a dry powder Beckman Coulter particle size analyzer.

16. The blood meal according to any one of claims 11-15, wherein the blood is obtained from animals, chosen from poultry, duck, swine, cattle, including cows, horses, goat, and sheep.

17. Use of the blood meal obtained according to any one of claims 1-10 or the blood meal according to any one of claims 11-16 as feed and/or feed additive, like in pet food or for aquaculture feed, or in cosmetics, as carrier and/or protein extender for pet food and feed palatants.

## Patentansprüche

1. Verfahren zur Herstellung von Blutmehl, umfassend die folgenden Schritte: (i) Bereitstellen einer wässrigen Mischung, umfassend Rohblut, vorzugsweise mit einem Feststoffgehalt zwischen 5 und 18 Gew.-%, und (ii) Erhöhen des Feststoffgehalts der Mischung, um eine Mischung mit einem Feststoffgehalt von 20 Gew.-% oder höher, vorzugsweise zwischen 20 und 80 Gew.-%, zu erhalten, und (iii) gleichzeitiges Trocknen und Mahlen der entstandenen Mischung in einer Luftwirbelmühle, um Blutmehl mit einer durchschnittlichen Partikelgröße (d50) zwischen 20 µm und 0,7 mm, einem d90 von unter 1 mm, gemessen mit Laserbeugung unter Verwendung eines Trockenpulver-Partikelgrößenanalysators von Beckman Coulter, und einer ilealen Verdaulichkeit, gemessen nach dem von Boison in Animal Feed Science Technology, 51, Seiten 29-43 (1995) und in Livestock Science, 309, Seiten 182-185 (2007), beschriebenen Verfahren, von 85 % oder höher, vorzugsweise von 87 % oder höher, und noch bevorzugter von 90 % oder höher zu erhalten.

2. Verfahren nach Anspruch 1, wobei die wässrige Mischung von Schritt (i) einem Koagulationsschritt, vorzugsweise unter Verwendung von Frischdampf, unterzogen wird und wobei anschließend ein erhöhter Feststoffgehalt durch Entfernen eines Teils des Wassers erhalten wird, um eine geronnene Blutmischung mit einem Feststoffgehalt zwischen 30 bis 70 Gew.-%, vorzugsweise 40 bis 60 Gew.-%, zu erhalten.

3. Verfahren nach Anspruch 1, wobei die wässrige Mischung Rohblut umfasst und wobei die Mischung einen Feststoffgehalt unter 18 Gew.-% oder unter 12 Gew.-% aufweist, wobei die Mischung gegebenenfalls homogenisiert wird und wobei der Feststoffgehalt durch Entfernung von Wasser und/oder durch Zugabe von Trockenblutmehl und/oder konzentriertem Blut während Schritt (ii) auf einen Feststoffgehalt von 30 Gew.-% oder mehr erhöht wird, wobei vorzugsweise mindestens ein Teil der Erhöhung des Feststoffgehalts durch Rückmischen von Trockenblutmehl in die Mischung erreicht wird.

4. Verfahren nach Anspruch 3, wobei die Menge des rückgemischten Blutmehls zwischen 2 Gew.-% und 90 Gew.-% (des getrockneten Materials), vorzugsweise zwischen 10 Gew.-% und 80 Gew.-%, beträgt.

5. Verfahren nach einem der vorhergehenden Ansprüche, wobei der Feststoffgehalt in Schritt (ii) um 10 Gew.-% oder mehr, vorzugsweise um 20 Gew.-% oder mehr, erhöht wird.

6. Verfahren nach einem der vorhergehenden Ansprüche, wobei das gleichzeitige Trocknen und Mahlen bei einer solchen Temperatur durchgeführt wird, dass das zu trocknende und zu mahlende Material bei einer Temperatur von 90 °C oder darunter, vorzugsweise von 80 °C oder darunter und noch
bevorzugter unter 60 °C bleibt.

7. Verfahren nach einem der vorhergehenden Ansprüche, wobei die Luftwirbelmühle eine Kammer mit geeigneten Einlässen und Auslässen für Produkt und Gasstrom/Gasströme umfasst, in der ein rotierendes Element mit Stapeln von Prallvorrichtungen montiert ist, wobei das rotierende Element mit hoher Geschwindigkeit rotieren kann, und wobei vorzugsweise die Innenwände des Stators mit Prallelementen ausgekleidet sind, wobei das rotierende Element mit einer Spitzengeschwindigkeit zwischen 35-250 m/s rotiert.

8. Verfahren nach einem der vorhergehenden Ansprüche, wobei die Luftwirbelmühle eine interne oder externe Sortiervorrichtung umfasst, die eine Rezirkulation von grobem Material in die Mühle ermöglicht.

9. Verfahren nach einem der vorhergehenden Ansprüche, wobei die Luftwirbelmühle mit einer Gasströmung, vorzugsweise Luft mit optional verringertem Sauerstoffgehalt, bei einer Temperatur zwischen 20 °C und 500 °C, vorzugsweise zwischen 20 °C und 450 °C, betrieben wird und wobei die Gasströmung zwischen 5 und 50 m3/h pro kg Futtermittel beträgt, wobei die Strömung eingestellt werden kann, um die Partikelgröße des trockenen Blutmehls zu beeinflussen, und wobei die Verweilzeit weniger als 10 Sekunden beträgt.

10. Verfahren nach einem der vorhergehenden Ansprüche, wobei das Blut von Tieren gewonnen wird, ausgewählt aus Geflügel, Enten, Schweinen, Vieh, einschließlich Kühen, Pferden, Ziegen und Schafen.

11. Koaguliertes Blutmehl mit einer durchschnittlichen Partikelgröße (d50) zwischen 20 µm und 0,7 mm, einem d90 von unter 1 mm, gemessen mit Laserbeugung unter Verwendung eines Trockenpulver-Partikelgrößenanalysators von Beckman Coulter, und einer ilealen Verdaulichkeit, gemessen nach dem von Boison in Animal Feed Science Technology, 51, S. 29-43, (1995) und in Livestock Science, 309: S.182-185 (2007) beschriebenen Verfahren, von 85% oder höher, vorzugsweise 87% oder höher und noch bevorzugter 90% oder höher, und einem Feuchtigkeitsgehalt von 10 Gew.-% oder weniger, vorzugsweise 5-8 Gew.-%.

12. Die Mahlzeit Blut nach Anspruch 11, wobei das trockenen Blutmehl einen bioverfügbar Lysingehalt hat, gemessen nach ISO 5510: 1984 von 92 % oder höher, vorzugsweise 94 % oder höher.

13. Blutmehl nach einem der Ansprüche 11 bis 12, wobei der Scheinbare Verdaulichkeitskoeffizient, gemessen nach dem in der Beschreibung des Trockenblutmehls an Forellen definierten Verfahren, 80 % oder höher, bezogen auf Rohprotein, vorzugsweise 85 % oder höher, und noch bevorzugter 87 % oder höher ist.

14. Blutmehl nach einem der Ansprüche 11 bis 13, wobei das Blutmehl eine durchschnittliche Partikelgröße (d50) zwischen 20 µm und 0,5 mm, gemessen durch Laserbeugung unter Verwendung eines Trockenpulver-Beckman-Coulter-Partikelgrößenanalysators, vorzugsweise zwischen 50 µm und 300 µm, aufweist.

15. Blutmehl nach einem der Ansprüche 11 bis 14, wobei das Blutmehl eine d90-Partikelgröße von 0,7 mm oder weniger aufweist, gemessen durch Laserbeugung unter Verwendung eines Trockenpulver-Beckman-Coulter-Partikelgrößenanalysators, und/oder wobei das Blutmehl eine d10-Partikelgröße von 10 µm oder mehr, vorzugsweise von 15 µm oder mehr, aufweist, gemessen durch Laserbeugung unter Verwendung eines Trockenpulver-Beckman-Coulter-Partikelgrößenanalysators.

16. Blutmehl nach einem der Ansprüche 11 bis 15, wobei das Blut von Tieren gewonnen wird, ausgewählt aus Geflügel, Ente, Schwein, Vieh, einschließlich Kühen, Pferden, Ziege und Schaf.

17. Verwendung des nach einem der Ansprüche 1 bis 10 gewonnenen Blutmehls oder des nach einem der Ansprüche 11 bis 16 gewonnenen Blutmehls als Futtermittel und/oder Futtermittelzusatzstoff, wie in Heimtierfutter oder für Aquakulturfutter, oder in Kosmetika, als Trägerstoff und/oder Proteinstreckmittel für Heimtierfutter und Futtermittelpalatanten.

## Revendications

1. Procédé de production de farine de sang comprenant les étapes suivantes consistant à (i) fournir un mélange aqueux comprenant du sang frais, ayant de préférence une teneur en matière sèche comprise entre 5 et 18 % en poids et (ii) augmenter la teneur en matière sèche du mélange pour obtenir un mélange ayant une teneur en matière sèche de 20 % en poids ou plus, de préférence entre 20 et 80 % en poids et (iii) sécher et broyer simultanément le mélange résultant dans un broyeur à turbulence d'air, pour obtenir de la farine de sang ayant une taille moyenne de particule (d50) entre 20 µm et 0,7 mm, une d90 inférieure à 1 mm telle que mesurée par diffraction laser à l'aide d'un analyseur de taille de particule Beckman Coulter de poudre sèche et une digestibilité iléale telle que mesurée selon la méthode décrite par Boison dans *Animal Feed Science Technology,* 51, pp.29-43 (1995) et dans *Livestock Science,* 309: pp. 182-185 (2007), de 85 % ou plus, de préférence de 87 % ou plus, et plus préférablement de 90 % ou plus.

2. Procédé selon la revendication 1, dans lequel le mélange aqueux de l'étape (i) est soumis à une étape de coagulation, de préférence à l'aide de vapeur vive, et dans lequel par la suite une teneur en matière sèche augmentée est obtenue par élimination d'une partie de l'eau pour obtenir un mélange de caillot de sang ayant une teneur en matière sèche comprise entre 30 et 70 % en poids, de préférence entre 40 et 60 % en poids.

3. Procédé selon la revendication 1, dans lequel le mélange aqueux comprend du sang frais et dans lequel le mélange a une teneur en matière sèche inférieure à 18 % en poids, ou inférieure à 12 % en poids, dans lequel le mélange est éventuellement homogénéisé et est augmenté en teneur en matière sèche en une teneur en matière sèche de 30 % en poids ou plus par élimination d'eau et/ou par addition de farine de sang sec et/ou de sang concentré au cours de l'étape (ii), de préférence au moins une partie de l'augmentation de la teneur en matière sèche est obtenue en remélangeant la farine de sang sec dans le mélange.

4. Procédé selon la revendication 3, dans lequel la quantité de farine de sang remélangée est comprise entre 2 % en poids et 90 % en poids (du matériau séché), de préférence entre 10 % en poids et 80 % en poids.

5. Procédé selon l'une quelconque des revendications précédentes, dans lequel la teneur en matière sèche dans l'étape (ii) est augmentée de 10 % en poids ou plus, de préférence de 20 % en poids ou plus.

6. Procédé selon l'une quelconque des revendications précédentes, dans lequel le séchage et le broyage sont effectués simultanément à une température telle que le matériau étant séché et broyé reste à une température de 90 °C ou moins, de préférence de 80 °C ou moins et plus préférablement inférieure à 60 °C.

7. Procédé selon l'une quelconque des revendications précédentes, dans lequel le broyeur à turbulence d'air comprend une chambre dotée d'entrées et de sorties appropriées pour le produit et l'écoulement ou les écoulements de gaz dans laquelle un élément rotatif est monté avec des empilements de dispositifs d'impact lequel élément rotatif pouvant tourner à grande vitesse , et dans lequel de préférence les parois internes du stator sont revêtues d'éléments d'impact, dans lequel l'élément rotatif tourne à une vitesse de pointe comprise entre 35 et 250 m/s.

8. Procédé selon l'une quelconque des revendications précédentes, dans lequel le broyeur à turbulence d'air comprend un dispositif de classification interne ou externe permettant la recirculation de matériau brute dans le broyeur.

9. Procédé selon l'une quelconque des revendications précédentes, dans lequel le broyeur à turbulence d'air fonctionne avec un écoulement de gaz, de préférence de l'air avec éventuellement une teneur en oxygène réduite, à une température comprise entre 20 °C et 500 °C, de préférence entre 20 °C et 450 °C et dans lequel l'écoulement de gaz est compris entre 5 et 50 m³/h par kg d'aliment, lequel écoulement peut être ajusté pour influencer la taille de particule de la farine de sang sec, et dans lequel le temps de séjour est inférieur à 10 secondes.

10. Procédé selon l'une quelconque des revendications précédentes, dans lequel le sang est obtenu à partir d'animaux, choisis parmi la volaille, le canard, le porc, le bétail, y compris les vaches, les chevaux, la chèvre et le mouton.

11. Farine de sang coagulé ayant une taille moyenne de particule (d50) entre 20 µm et 0,7 mm, une d90 inférieure à 1 mm telle que mesurée par diffraction laser à l'aide d'un analyseur de taille de particule Beckman Coulter de poudre sèche et une digestibilité iléale, telle que mesurée selon la méthode décrite par Boison dans *Animal Feed Science Technology,* 51, pp.29-43 (1995) et dans *Livestock Science,* 309: pp. 182-185 (2007), de 85 % ou plus, de préférence de 87 % ou plus et même plus préférablement de 90 % ou plus, et une teneur en humidité de 10 % en poids ou moins, de préférence de 5 à 8 % en poids.

12. Farine de sang selon la revendication 11, dans laquelle la farine de sang sec a une teneur en lysine biodisponible telle que mesurée selon ISO 5510 : 1984 de 92 % ou plus, de préférence de 94 % ou plus.

13. Farine de sang selon l'une quelconque des revendications 11 à 12, dans laquelle le coefficient de digestibilité apparente tel que mesuré selon le procédé défini dans la description de la farine de sang sec sur de la truite est de 80 % ou plus sur la base de protéine brute, de préférence de 85 % ou plus, et plus préférablement de 87 % ou plus.

14. Farine de sang selon l'une quelconque des revendications 11 à 13, dans laquelle la farine de sang a une taille moyenne de particule (d50) comprise entre 20 µm et 0,5 mm mesurée par diffraction laser à l'aide d'un analyseur de taille de particule Beckman Coulter de poudre sèche, de préférence comprise entre 50 µm et 300 µm.

15. Farine de sang selon l'une quelconque des revendications 11 à 14, dans laquelle la farine de sang a une taille de particule d90 de 0,7 mm ou moins mesurée par diffraction laser à l'aide d'un analyseur de taille de particule Beckman Coulter de poudre sèche, et/ou la farine de sang ayant une taille de particule d10 de 10 µm ou plus, de préférence de 15 µm ou plus, telle que mesurée par diffraction laser à l'aide d'un analyseur de taille de particule Beckman Coulter de poudre sèche.

16. Farine de sang selon l'une quelconque des revendications 11 à 15, dans laquelle le sang est obtenu à partir d'animaux, choisis parmi la volaille, le canard, le porc, le bétail, y compris les vaches, les chevaux, la chèvre et le mouton.

17. Utilisation de la farine de sang obtenue selon l'une quelconque des revendications 1 à 10 ou de la farine de sang selon l'une quelconque des revendications 11 à 16 en tant qu'aliment et/ou additif alimentaire, comme dans les aliments pour animaux de compagnie ou pour les aliments piscicoles, ou dans la cosmétique, en tant que protéine porteuse et/ou succédané protéique pour les aliments pour animaux de compagnie et les sapidités alimentaires.
